# EUROPEAN PATENT SPECIFICATION

(11) **EP 1 298 202 B1**
(45) Date of publication and mention of the grant of the patent: **06.10.2010**
(21) Application number: 02018736.5
(22) Date of filing: 27.10.1993
(51) Int. Cl.: C12N 5/0797

(54) **Biological factors and neural stem cells**
Biologische Faktoren und neuronale Stammzellen
Facteurs biologiques et cellules souches neurales

(30) Priority: 28.10.1992 US 967622
(43) Date of publication of application: 02.04.2003
(62) Divisional of application: 93923994.3
(73) Proprietor: NeuroSpheres Holdings Ltd., Calgary, Alberta T2L 2K7 (CA)
(72) Inventor: Weiss, Samuel, Calgary, Alberta T2L 1A6 (CA); Reynolds, Brent A., Calgary, Alberta T3B 2V6 (CA); Hammang, Joseph P., Barrington, RI 02806 (US); Baetge, E. Edward, Barrington, RI 02806 (US)
(74) Representative: Crump, Julian Richard John

(56) References cited:
- WO-A-89/03872
- WO-A-91/02003
- WO-A-93/01275
- CATTANEO ET AL: "PROLIFERATION AND DIFFERENTIATION OF NEURONAL STEM CELLS REGULATED BY NERVE GROWTH FACTOR" NATURE, vol. 347, 25 October 1990 (1990-10-25), pages 762-765, XP002911519 LONDON,GB
- REYNOLDS ET AL: "GENERATION OF NEURONS AND ASTROCYTES FROM ISOLATED CELLS OF THE ADULT MAMMALIAN CENTRAL NERVOUS SYSTEM" SCIENCE, vol. 255, 27 March 1992 (1992-03-27), pages 1707-1710, XP000561246 WASHINGTON D.C.,USA
- GODFRAIND ET AL: "IN VIVO ANALYSIS OF GLIAL CELL PHENOTYPES DURING A VIRAL DEMYELINATING DISEASE IN MICE" THE JOURNAL OF CELL BIOLOGY, vol. 109, no. 5, November 1989 (1989-11), pages 2405-2416, XP001165636 NEW YORK,USA

## Description

### FIELD OF THE INVENTION

The present invention is directed to the culturing, growth and differentiation of neural stem cells using various biological factors. More particularly, the invention is related to an in vitro method for increasing the number of precursor cells that differentiate into a particular phenotype by exposing the cells to specific biological factors or combinations thereof.

### SUMMARY OF THE INVENTION

An in vitro method of preparing differentiated cells from precursor cells is described. Specifically, the method is a method of preparing differentiated cells from neural stem cells comprising the steps of:
a) proliferating at least one isolated neural stem cell capable of producing progeny that are capable of differentiating into neurons, astrocytes, and oligodendrocytes in suspension in a serum-free culture medium comprising epidermal growth factor (EGF) as a mitogen that proliferates at least one stem cell to produce neural precursor cells, and
b) differentiating the neural precursor cells by removing the mitogen and culturing said neural precursor cells in an EGF-free differentiation-inducing culture medium to produce differentiated cells, said medium being prepared with exogenous growth factor selected from the group consisting of CNTT, bFGF, BDNF and retinoic acid, wherein CNTF signifies ciliary neurotrophic factor, bFGF signifies basic fibroblast growth factor, and BDNF signifies brain-derived neurotrophic factor.

Other embodiments of the invention are recited in claims 2-9.

### BACKGROUND OF THE INVENTION

The development of the nervous system begins at an early stage of fetal development. Neurogenesis, the generation of new neurons, is complete early in the postnatal period. However, the synaptic connections involved in neural circuits are continuously altered throughout the life of the individual, due to synaptic plasticity and cell death.

The first step in neural development is cell birth, which is the precise temporal and spatial sequence in which precursor or progenitor cells proliferate and differentiate. Proliferating cells will give rise to neuroblasts, glioblasts and stem cells.

The second step is a period of cell type differentiation and migration when progenitor cells become neurons and glial cells and migrate to their final positions. Cells which are derived from the neural tube give rise to neurons and glia of the central nervous system (CNS), while cells derived from the neural crest give rise to the cells of the peripheral nervous system (PNS). Certain factors present during development, such as nerve growth factor (NGF), promote the growth of neural cells. NGF is secreted by cells of the neural crest and stimulates the sprouting and growth of the neuronal axons.

The third step in development occurs when cells acquire specific phenotypic qualities, such as the expression of particular neurotransmitters. At this time, neurons also extend processes which synapse on their targets. Neurons do not divide subsequent to differentiation.

Finally, selective cell death occurs, wherein the degeneration and death of specific cells, fibers and synaptic connections "fine-tune" the complex circuitry of the nervous system. This "fine-tuning" continues throughout the life of the host. Later in life, selective degeneration due to aging, infection and other unknown etiologies can lead to neurodegenerative diseases.

Recently, the concept of neurological tissue grafting has been applied to the treatment of neurological diseases such as Parkinson's Disease. Neural grafts may avert the need not only for constant drug administration, but also for complicated drug delivery systems which arise due to the blood-brain barrier. However, there are limitations to this technique. First, cells used for transplantation which carry cell surface molecules of a differentiated cell from another host can induce an immune reaction in the host. In addition, the cells must be at a stage of development where they are able to form normal neural connections with neighboring cells. For these reasons, initial studies on neurotransplantation centered on the use of fetal cells. Perlow, et al. describe the transplantation of fetal dopaminergic neurons into adult rats with chemically induced nigrostriatal lesions in "Brain grafts reduce motor abnormalities produced by destruction of nigrostriatal dopamine system, "Science 204:643-647 (1979). These grafts showed good survival, axonal outgrowth and significantly reduced the motor abnormalities in the host animals. Lindvall et al., in "Grafts of fetal dopamine neurons survive and improve motor function in Parkinson's Disease," Science 257:574:577 (1990), showed that neural transplantation of human fetal mesencephalic dopamine neurons can restore dopamine synthesis and storage, and reduce rigidity and bradykinesia in patients suffering from Parkinson's disease. Freed, et al. in "Transplantation of human fetal dopamine cells for Parkinson's Disease, " Arch. Neurol. 47:505-512 (1990) also show improvement in a patient who received a fetal transplant.

The above references disclose that mammalian fetal brain tissue has good survival characteristics upon immediate transplantation. The increased survival capability of fetal neurons is thought to be due to the reduced susceptibility of fetal neurons to anoxia than adult neurons, and also to the lack of cell surface markers on fetal cells whose presence may lead to the rejection of grafted tissue from adults. However, although the brain is considered an immunologically privileged site, some rejection of fetal tissue can occur. Therefore, the ability to use fetal tissue is limited, not only due to tissue rejection of the fetal tissue isolated from another host, and because of the resultant need for immunosuppressant drugs, but also due to ethical problems in obtaining fetal tissue. However, neonatal brain tissue possesses limited capacity for survival and adult mammalian CNS neurons generally do not survive transplantation into the brain.

Although adult CNS neurons are not good candidates for neurotransplantation, neurons from the adult peripheral nervous system (PNS) have been shown to survive transplantation, and to exert neurotrophic and gliotrophic effects on developing host neural tissue. One source of non-CNS neural tissue for transplantation is the adrenal medulla. Adrenal chromaffin cells originate from the neural crest like PNS neurons, and receive synapses and produce carrier and enzyme proteins similar to PNS neurons. Although these cells function in an endocrine manner in the intact adrenal medulla, in culture these cells lose their glandular phenotype and develop neural features in culture in the presence of certain growth factors and hormones (Notter, et al., "Neuronal properties of monkey adrenal medulla in vitro," Cell Tissue Research 244:69-76 [1986]). When grafted into mammalian CNS, these cells survive and synthesize significant quantities of dopamine which can interact with dopamine receptors in neighboring areas of the CNS.

In U.S. Patent No. 4,980,174, transplantation of monoamine-containing cells isolated from adult rat pineal gland and adrenal medulla into rat frontal cortex led to the alleviation of learned helplessness, a form of depression in the host. In U.S. Patent No. 4,753,635, chromaffin cells and adrenal medullary tissue derived from steers were implanted into the brain stem or spinal cord of rats and produced analgesia when the implanted tissue or cell was induced to release nociceptor interacting substances (i.e. catecholamines such as dopamine). Adrenal medullary cells have been autologously grafted into humans, and have survived, leading to mild to moderate improvement in symptoms (Watts, et al., "Adrenal-caudate transplantation in patients with Parkinson's Disease (PD): 1-year follow-up," Neurology 39 Suppl 1:127 [1989]; Hurtig et al., "Post-mortem analysis of adrenal-medulla-to-caudate autograft in a patient with Parkinson's Disease," Annals of Neurology 25:607-614 [1989]). However, adrenal cells do not obtain a normal neural phenotype, and are therefore probably of limited use for transplants where synaptic connections must be formed.

Another source of tissue for neurotransplantation is from cell lines. Cell lines are immortalized cells which are derived either by transformation of normal cells with an oncogene (Cepko, "Immortalization of neural cells via retrovirus-mediated oncogene transduction," Ann. Rev. Neurosci. 12:47-65 [1989]) or by the culturing of cells with altered growth characteristics in vitro (Ronnett, et al., "Human cortical neuronal cell line: Establishment from a patient with unilateral megalencephaly," Science 248:603-605 [1990]). Such cells can be grown in culture in large quantities to be used for multiple transplantations. Some cell lines have been shown to differentiate upon chemical treatment to express a variety of neuronal properties such as neurite formation, excitable membranes and synthesis of neurotransmitters and their receptors. Furthermore, upon differentiation, these cells appear to be amitotic, and therefore non-cancerous. However, the potential for these cells to induce adverse immune responses, the use of retroviruses to immortalize cells, the potential for the reversion of these cells to an amitotic state, and the lack of response of these cells to normal growth-inhibiting signals make cell lines less than optimal for widespread use.

O-2A cells are glial progenitor cells which give rise in vitro only to oligodendrocytes and type II astrocytes. Cells which appear by immunostaining in vivo to have the O-2A phenotype have been shown to successfully remyelinate demyelinated neurons in vivo. Godfraind et al., J. Cell Biol. 109:2405-2416 (1989). Injection of a large number of O-2A cells is required to adequately remyelinate all targeted neurons in vivo, since it appears that O-2A cells (like other glial cell preparations) do not continue to divide in situ. Although O-2A progenitor cells can be grown in culture, currently the only available isolation technique employs optic nerve as starting material. This is a low yield source, which requires a number of purification steps. There is an additional drawback that O-2A cells isolated by the available procedures are capable of only a limited number of divisions. Raff Science 243:1450-1455 (1989).

Transformed O-2A cell lines are unsuitable for transplantation due to the fact that the transformation process leads to a genetically (oncogene) controlled cell division as opposed to primary cell lines or neural stem or progenitor cells where regulation of division is at an epigenetic level. Additional potential problems include instability of cell lines over long periods of time, and aberrant patterns of differentiation or responses to growth factors. Goldman Trends Neuro. Sci. 15:359-362 (1992).

In Science vol. 255, March 1992, pages 1707-1710, striata of adult mouse were dissociated and plated in serum-free medium containing EGF without supplementary substrate or adhesion factors. After 6-8 days in vitro (DIV), a detached sphere of proliferating cells with characteristics of neuroepithelial stem cells had established. Upon dissociation of the cells from the spheres and replating in EGF, proliferation as nestin⁺ cells occurred. Upon transfer of 6-8 DIV spheres to poly-L-ornithine coated substrate and continued presence of EGF, differentiated cells were determined as present after 21 DIV.

The inability in the prior art of the transplant to fully integrate into the host tissue, and the lack of availability of cells in unlimited amounts from a reliable source for grafting are, perhaps, the greatest limitations of neurotransplantation.

Therefore, in view of the aforementioned deficiencies attendant with prior art methods of neural cell culturing and transplantation, it should be apparent that there still exists a need in the art for a reliable source of unlimited numbers of cells for neurotransplantation, which are capable of differentiating into neurons and glial cells.

Accordingly, it is an object of this invention to provide a method for providing a reliable source of epigenetically regulated cells for transplantation, which are capable of differentiating into neurons and glial cells.

It is another object of the invention to provide a method for influencing the differentiation of precursor cells using specific growth factors.

These and other objects and features of the invention will be apparent to those skilled in the art from the following detailed description and appended claims.

None of the foregoing references is believed to disclose the present invention as claimed and is not presumed to be prior art. The references are offered for the purpose of background information.

### DETAILED DESCRIPTION OF THE INVENTION

### Definitions

The term "stem cell" refers to an undifferentiated cell which is capable of proliferation and giving rise to more stem cells having the ability to generate a large number of progenitor cells that can in turn give rise to differentiated, or differentiable daughter cells.

The term "neural stem cell" (NSC) refers to the stem cells of the instant invention, the progeny of which under appropriate culturing conditions, include both glial and neuronal progenitor cells.

The term "progenitor cells" refers to the undifferentiated cells of the instant invention, derived from neural stem cells, the progeny of which may, under appropriate conditions, include glial and/or neuronal progenitor cells.

The term "oligodendrocyte" refers to a differentiated glial cell which forms the myelin surrounding axons in the central nervous system (CNS). Oligodendrocytes are of the phenotype galactocerebroside (+), myelin basic protein (+), and glial fibrillary acidic protein (-) [GalC(+), MBP(+), GFAP(-)].

The term "neuron" refers to a cell having the phenotype neurospecific enolase (+) or neurofilament (+) [NSE(+) or NF (+)].

The term "type I astrocyte" refers to a differentiated glial cell type with a flat protoplasmic/fibroblast-like morphology that is GFAP(+), A2B5 (-), GalC(-), and MBP(-).

The term "type II astrocyte" refers to a differentiated glial cell displaying a stellate process-bearing morphology of the phenotype GFAP(+), A2B5(+), GalC(-), and MBP(-).

The term "neuronal progenitor cells" refers to cells which produce daughter cells which under the appropriate conditions become or give rise to neurons.

The term "oligodendrocyte precursor cells" refers to cells which give rise to oligodendrocytes. Oligodendrocyte precursor cells can have the phenotype A2B5(+), O4(+)/GalC(-), MBP(-) and GFAP (-) [but are not limited to this phenotype].

The term "neurosphere" refers to a cluster of cells derived from neural stem cells and cultured in vitro. At least some of the cells are of the nestin (+) phenotype. The cluster is comprised of stem cells and/or progenitor cells and may or may not include differentiated cells.

The term "precursor cells" refers to the living cells of the instant invention that are derived from neural stem cells proliferated in a culture medium containing a growth factor or factors, and includes both progenitor and stem cells. Precursor cells typically grow in the form of neurospheres, but may exhibit different growth patterns depending upon culture conditions.

The term "growth factor" refers to a protein, peptide or other molecule having a growth, proliferative, differentiative, or trophic effect.

The term "donor" refers to the human or animal which is the source of the neural stem cells used in the instant invention.

### Description of the Preferred Embodiments

### Phenotypical characteristics

Neural stem cells (NSCs) have been reported and their potential use described. (Reynolds and Weiss, Science 255:1707 (1992)). It has been shown that NSCs give rise to neuroblasts (Reynolds and Weiss, Restorative Neurology & Neuroscience 4:208 (1992)). It is now known that NSCs also give rise to major macroglial cell types (astrocytes and oligodendrocytes).

Neural stem cells can be isolated and cultured by the method of Reynolds and Weiss (supra). In brief, the epidermal growth factor (EGF)-responsive stem cell, when grown in a defined serum-free medium, and in the presence of a mitogen such as EGF or the like, is induced to divide giving rise to a cluster of undifferentiated cells. The clusters of cells are not immunoreactive for GFAP, neurofilament (NF), neuron-specific enolase (NSE) or MBP. However, precursor cells within the cluster are immunoreactive for nestin, an intermediate filament protein found in undifferentiated CNS cells. The nestin marker was characterized by Lehndahl et al., Cell 60:585-595 (1990). The mature phenotypes associated with the four cell types which may be differentiated from the progeny of the precursor cells are predominantly negative for the nestin phenotype.

In the continued presence of a mitogen such as EGF or the like, precursor cells within the neurosphere continue to divide resulting in an increase in the size of the neurosphere and the number of undifferentiated cells [nestin(+), GFAP(-), NF(-), NSE (-), MBP (-)]. At this stage, the cells are non-adherent and tend to form the free-floating clusters characteristic of neurospheres. However, culture conditions may be varied so that while the precursor cells still express the nestin phenotype, they do not form the characteristic neurospheres. After removal of the mitogen, the cells adhere to the substrate (poly-ornithine-treated plastic or glass), flatten, and begin to differentiate into neurons and glial cells. At this stage the culture medium may contain serum such as 0.5-1.0% fetal bovine serum (FBS). Within 2-3 days, most or all of the precursor cells begin to lose immunoreactivity for nestin and begin to express intermediate filaments specific for neurons or for astrocytes as indicated by immunoreactivity to NF or GFAP respectively.

The identification of neurons is accomplished using immunoreactivity for neuron-specific enolase (NSE) and the neurofilament proteins tau-1 and MAP-2. Because these markers are highly reliable, they will continue to be useful for the primary identification of neurons, however neurons can also be identified based on their specific neurotransmitter phenotype.

Using dual-label immunofluorescence and immunoperoxidase methods, differentiated neurosphere cultures can be analyzed for the expression of neurotransmitters, or in some cases for the enzymes responsible for the neurotransmitter synthesis. Alternatively, in situ hybridization histochemistry can be performed using cDNA or RNA probes specific for the peptide neurotransmitter or the neurotransmitter synthesizing enzyme mRNAs. These techniques can be combined with immunocytochemical methods to enhance the identification of specific phenotypes. If necessary, the antibodies and molecular probes discussed above can be applied to western and northern blot procedures respectively to aid in the cell identification.

Alternatively, high performance liquid chromatography (HPLC) methods can be used in phenotype identification. HPLC is particularly useful for the identification of a number of small peptide neurotransmitters and catecholamine and indoleamine neurotransmitters. These techniques are highly sensitive and can be used in large-scale screening paradigms requiring relatively small sample volumes.

In addition to the presence of neurons and astrocytes, a large number of cells that do not express either intermediate filaments specific for neurons or for astrocytes, begin to express markers specific for oligodendrocytes in a correct temporal fashion. That is, the cells first become immunoreactive for 04 (a cell surface antigen), galactocerebroside (GalC, a myelin glycolipid) and finally, myelin basic protein (MBP). These cells also possess a characteristic oligodendrocyte morphology.

The present invention provides a method of influencing the relative proportion of these differentiated cell types by the addition of exogenous growth factors during the differentiation stage of the precursor cells. By using dual-label immunofluorescence and immunoperoxidase methods with various neuronal- and glial-specific antibodies, the effect of the exogenous growth factors on the differentiating cells can be determined.

The biological effects of growth and trophic factors is generally mediated through binding to cell surface receptors. The receptors for a number of these factors have been identified and antibodies and molecular probes for specific receptors are available. Neural stem cells can be analyzed for the presence of growth factor receptors at all stages of differentiation. In many cases, the identification of a particular receptor will define the strategy to use in further differentiating the cells along specific developmental pathways with the addition of exogenous growth or trophic factors.

Exogenous growth factors can be added alone or in various combinations. They can also be added in a temporal sequence (i.e. exposure to a first growth factor influences the expression of a second growth factor receptor, Neuron 4:189-201 (1990). Among the growth factors and other molecules that can be used to influence the differentiation of precursor cells in vitro are acidic and basic fibroblast growth factor (aFGF & bFGF), ciliary neurotrophic factor (CNTF), nerve growth factor (NGF), brain-derived neurotrophic factor (BDNF), neurotrophin 3 (NT3), neurotrophin 4 (NT4), interleukins, leukemia inhibitory factor (LIF), cyclic adenosine monophosphate (cAMP), forskolin, tetanus toxin, high levels of potassium (High K⁺), amphiregulin, transforming growth factor-alpha (TGF-α), transforming growth factor betas (TGF-βs), insulin-like growth factors, dexamethasone (glucocorticoid hormone), isobutyl 3-methylxanthine (IBMX), somatostatin, growth hormone, retinoic acid, and platelet-derived growth factor (PDGF). These and other growth factors and molecules will find use in the present invention.

The differentiation of precursor cells may also be induced by various substrates in addition to poly-L-ornithine such as collagen, fibronectin, laminin, matrigel etc.

### Examples

### Example 1

### Propagation of precursor cells

Fetal day 14 (E14) CD₁ albino mice (Charles River) were decapitated and the brain and striata removed using sterile procedure. The tissue was mechanically dissociated with a fire-polished Pasteur pipette into serum-free medium composed of a 1:1 mixture of Dulbecco's modified Eagle's medium (DMEM) and F-12 nutrient mixture (Gibco). The cells were centrifuged at 800 r.p.m. for 5 minutes, the supernatant aspirated, and the cells resuspended in DMEM/F-12 medium for counting.

The cells were suspended in a serum-free medium, hereinafter referred to as "complete medium", composed of DMEM/F-12 (1:1) which included glucose (0.6%), glutamine (2 mM), sodium bicarbonate (3 mM), HEPES (4-[2-hydroxyethyl]-1-piperazineethanesulfonic acid) buffer (5 mM) and a defined hormone mix and salt mixture (to replace serum) that included insulin (25 µg/ml), transferrin (100 µg/ml), progesterone (20 nM), putrescine (60 µM), and selenium chloride (30 nM) (all from Sigma except glutamine [Gibco]). In addition, the medium contained 16-20 ng/ml EGF (purified from mouse submaxillary, Collaborative Research) or TGFα (human recombinant, Gibco). The cells were plated at 0.2 x 10⁶ cells/ml into 75 cm² tissue culture flasks (Corning) with no substrate pre-treatment and housed in an incubator at 37°C, 100% humidity, 95% air/5% CO₂.

When the cells were proliferated, within the first 48 hours and by 3-4 days in vitro (DIV), they formed small clusters, known as neurospheres, that lifted off the substrate between 4-6 DIV.

After 7 DIV, the neurospheres were removed, centrifuged at 400 r.p.m. for 2-5 minutes, and the pellet was mechanically dissociated into individual cells with a fire-polished glass Pasteur pipet in 2 mls of complete medium.

1 x 10⁶ cells were replated into a 75 cm² tissue culture flask with 20 mls of the EGF-containing complete medium. The proliferation of the stem cells and formation of new neurospheres was reinitiated. This procedure can be repeated every 6-8 days.

### Example 2

### Differentiation of neurospheres

Neurospheres were differentiated using the following paradigms. The neurospheres used for each paradigm were generated as outlined in Example 1. All the neurospheres used were passed at least once prior to their differentiation.

### Paradigm 1 -- Rapid differentiation of neurospheres

Six to 8 days after the first passage, the neurospheres were removed and centrifuged at 400 r.p.m. The EGF-containing supernatant was removed and the pellet suspended in EGF-free complete medium containing 1% fetal bovine serum (FBS).

Neurospheres (approximately 0.5-1.0 x 10⁶ cells/well) were plated on poly-L-ornithine-coated (15 µg/ml) glass coverslips in 24 well Nuclon (1.0 ml/well) culture dishes. After 24 hours in culture, the coverslips were transferred to 12 well (Costar) culture dishes containing complete medium containing 0.5% FBS. The medium was changed every 4-7 days. This differentiation procedure is referred to as the "Rapid Differentiation Paradigm" or RDP.

### Paradigm 2 -- Differentiation of dissociated neurospheres

Six to 8 days after the first passage, the neurospheres were removed and centrifuged at 400 r.p.m. The EGF-containing media was removed and the pellet was suspended in EGF-free complete medium containing 1% FBS. The neurospheres were mechanically dissociated into single cells with a fire-polished Pasteur pipette and centrifuged at 800 r.p.m. for 5 minutes. Between 0.5 x 10⁶ and 1.0 x 10⁶ cells were plated on poly-L-ornithine-coated (15 µg/ml) glass coverslips in 24 well Nuclon (1.0 ml/well) culture dishes. The EGF-free culture medium containing 1% FBS was changed every 4-7 days.

### Paradigm 3 -- Differentiation of single neurospheres

Neurospheres were washed free of EGF by serial transfers through changes of EGF-free medium. A single neurosphere was plated onto poly-L-ornithine-coated (15 µg/ml) glass coverslips in a 24-well plate. The culture medium used was complete medium with or without 1% FBS. The medium was changed every 4-7 days.

### Paradigm 4 -- Differentiation of single dissociated neurospheres

Neurospheres were washed free of EGF by serial transfers through changes of EGF-free medium. A single neurosphere was mechanically dissociated in a 0.5 ml Eppendorf centrifuge tube and all the cells were plated onto a 35 mm culture dish. Complete medium was used with or without 1% FBS.

### Example 3

### Effect of growth factors on neurosphere differentiation

The effects of CNTF, bFGF, BDNF, and Retinoic Acid on neurosphere differentiation were tested using the differentiation paradigms set forth in Example 2.

### CNTF

The effect of CNTF was assayed in paradigms 1 and 3. For both paradigms, CNTF was added either at the beginning of the experiment at a concentration of 10 ng/ml or daily at a concentration of 1 ng/ml.

In paradigm 1, the addition of CNTF increased the number of neuron-specific enolase (NSE)-immunoreactive cells in addition to the number of tau-1-immunoreactive cells, suggesting that CNTF has an effect on the proliferation, survival, or differentiation of neurons. Preliminary testing with antibodies recognizing the neurotransmitters GABA and Substance P suggest that there is no increase in the number of cells containing these proteins. This suggests that a different neuronal phenotype is being produced.

Three different antibodies directed against 04, galactocerebroside (GalC) and myelin basic protein (MBP) were used to study the effect of CNTF on the oligodendrocytes of paradigm 1. CNTF had no effect on the number of O4(+) cells, but there was an increase in the number of GalC(+) and MBP(+) cells compared with the control. Thus it appears that CNTF plays a role in the maturation of oligodendrocytes.

In one experiment, the neurospheres were differentiated as outlined in paradigm 1 except that serum was never added to the culture medium. While the effect of CNTF on neurons and oligodendrocytes was not as apparent as in the presence of serum, there was an increase in the proliferation of flat, protoplasmic astrocytes. Hence, CNTF will affect astrocyte differentiation in various culture conditions.

In paradigm 3, the addition of CNTF resulted in an increase in the number of NSE(+) cells.

### BDNF

The effect of BDNF was tested using Paradigm 3. There was an increase in the number of NSE(+) neurons per neurosphere. Additionally, there was an increase in the neuronal branching and the migration of the neurons away from the sphere.

### bFGF

The effect of bFGF was tested using paradigms 2 and 4. In paradigm 2, 20 ng/ml of bFGF was added at the beginning of the experiment and cells were stained 7 days later. bFGF increased the number of GFAP(+) cells and the number of NSE(+) cells. This suggests that bFGF has a proliferative or survival effect on the neurons and astrocytes.

In paradigm 4, 20 ng/ml of bFGF was added at the beginning of the experiment and assayed 7-10 days later. bFGF induced the proliferation of precursor cells generated by the EGF-responsive stem cell. It induced two different cell types to divide, neuroblasts and bipotential progenitor cells. The neuroblast produced, on average, 6 neurons while the bipotential cell produced approximately 6 neurons and a number of astrocytes.

In previous studies, it was found that when plated at low density (2500 cells/cm²), addition of EGF up to 7 days in vitro (DIV) could initiate proliferation of the stem cell, but not if applied after 7 DIV. Striatal cells (E14, 2500 cell/cm²) were plated in the absence or presence of 20 ng/ml of bFGF. After 11 DIV, cultures were washed and medium containing 20 ng/ml of EGF was added. After 4-5 DIV, in cultures that were primed with bFGF, greater than 70% of the wells examined contained clusters of proliferating cells that developed into colonies with the morphologic and antigenic properties of the EGF-generated cells. Cultures that had not been primed with bFGF showed no EGF-responsive proliferation. These findings suggest that the EGF-responsive stem cells possess bFGF receptors that regulate its long term survival.

### Retinoic acid

The effect of retinoic acid at 10⁻⁷M was tested using paradigm 1. There was an increase in the number of NSE(+) and tau-1(+) cells, suggesting that retinoic acid increases the number of neurons.

### Example 4

### Screening for the trkB receptor on neural stem cells

The expression of the trk family of neurotrophin receptors in EGF-generated neurospheres was examined by northern blot analysis. Total mRNA was isolated from mouse and rat striatal EGF-generated neurospheres. Both rat and mouse neurospheres expressed high levels of trkB receptor mRNA, but did not express trk nor trkC mRNA. In preliminary experiments, single EGF-generated mouse neurospheres were plated on poly-L-ornithine coated glass coverslips and cultured in the absence or presence of 10ng/ml of BDNF. When examined after 14-28 days in vitro, neurospheres plated in the presence of BDNF contained NSE(+) cells with extensive and highly branched processes; well-developed NSE(+) cells were not observed in the absence of BDNF. Activation of the trkB receptor on EGF-generated neurospheres may enhance differentiation, survival of and/or neurite outgrowth from newly generated neurons.

### Example 5

### Screening for the GAP-43 membrane phosphoprotein on neural stem cells

Growth-associated protein (GAP-43) is a nervous system-specific membrane phosphoprotein which is down-regulated during development. Originally, GAP-43 was though to be neuron-specific, however, recent reports indicate that this protein may be at least transiently expressed during development in some astrocytes, oligodendrocytes and in Schwann cells. At present, the role of GAP-43 in macroglia is not known. The transient expression of GAP-43 in glial cells generated from the EGF-responsive stem cells derived from embryonic and adult murine striatum was investigated. Glial cell (astrocyte and oligodendrocyte) differentiation was induced by plating precursor cells in a medium containing 1% FBS with no EGF. The cells were then probed with specific antibodies for GAP-43, nestin, GFAP, 04, and GalC. In order to identify cells expressing GAP-43, the antibodies were pooled in various combinations using dual-label immunofluorescence methods.

During the first two days post plating, there was a low to moderate level of GAP-43 expression in almost all cells (flat, bipolar and stellate), but by 3-4 days post-plating, the level of GAP-43 expression became restricted to the bipolar and stellate cells. At 4 days the majority of GAP-43-expressing cells co-labelled with the oligodendrocyte markers 04 and GalC although GFAP and GAP-43 was co-expressed in a number of cells. At one week post-plating however, essentially all of the GFAP-expressing astrocytes no longer expressed GAP-43 while the majority of the 04 and GalC-expressing cells continued to express GAP-43. At 7-10 days, these oligodendrocytes began to express MBP and lose the expression of GAP-43. The EGF-responsive stem cells may represent a useful model system for the study of the role of GAP-43 in glial and neuronal development.

## Claims

1. A method of preparing differentiated cells from neural stem cells comprising the steps of:
a) proliferating at least one isolated neural stem cell capable of producing progeny that are capable of differentiating into neurons, astrocytes, and oligodendrocytes in suspension in a serum-free culture medium comprising epidermal growth factor (EGF) as a mitogen that proliferates at least one stem cell to produce neural precursor cells, and
b) differentiating the neural precursor cells by removing the mitogen and culturing said neural precursor cells in an EGF-free differentiation-inducing culture medium to produce differentiated cells, said medium being prepared with exogenous growth factor selected from the group consisting of CNTT, bFGF, BDNF and retinoic acid, wherein CNTF signifies ciliary neurotrophic factor, bFGF signifies basic fibroblast growth factor, and BDNF signfies brain-derived neurotrophic factor.

2. The method of claim 1, wherein the differentiation-inducing culture medium contains serum.

3. The method of claim 1, wherein the differentiated cells are neurons and the differentiation-inducing growth factor is ciliary neurotrophic factor.

4. The method of claim 1, wherein the differentiated cells are mature oligodendrocytes and the differentiation-inducing growth factor is ciliary neurotrophic factor.

5. The method of claim 1, wherein the differentiated cells are astrocytes, the differentiation-inducting growth factor is ciliary neurotrophic factor, and the differentiation-inducing culture medium is substantially free of serum.

6. The method of claim 1, wherein the differentiated cells are neurons and the differentiation-inducing growth factor is brain-derived neurotrophic factor.

7. The method of claim 1, wherein the differentiated cells are neurons and the differentiation-inducing growth factor is retinoic acid.

8. The method of claim 1, wherein the differentiated cells are neurons and the difterentiation-inducing growth factor is bFGF.

9. The method of any one of the preceding claims, wherein proliferating the at least one previously obtained mammalian neural stem cell in step a) further comprises the steps of:
i) maintaining the obtained neural stem cells in a first proliferation-inducing culture medium containing bFGF; and
ii) proliferating the maintained neural stem cells in a second proliferation-inducing culture medium containing EGF and bFGF to produce neural precursor cells.

## Patentansprüche

1. Verfahren zur Herstellung differenzierter Zellen aus neuralen Stammzellen, folgende Schritte aufweisend:
a) Vermehren mindestens einer isolierten neuralen Stammzelle, die in der Lage ist, Abkömmlinge hervorzubringen, die in der Lage sind, in Suspension in einem serumfreien Kulturmedium, das epidermalen Wachstumsfaktor (EGF) aufweist als ein Mitogen, das mindestens eine Stammzelle proliferiert, um neurale Vorläuferzellen hervorzubringen, zu Neuronen, Astrozyten und Oligodendrozyten zu differenzieren, und
b) Differenzieren der neuralen Vorläuferzellen durch Entfernen des Mitogens und Kultivieren der neuralen Vorläuferzellen in einem EGF-freien differenzierungsinduzierenden Kulturmedium, um differenzierte Zellen hervorzubringen, wobei das Medium hergestellt wird mit exogenem Wachstumsfaktor, der ausgewählt wird aus der Gruppe, die aus CNTF, bFGF, BDNF und Retinsäure besteht, wobei CNTF ciliärer neurotropher Faktor bedeutet, bFGF basischer Fibroblastenwachstumsfaktor bedeutet, und BDNF aus dem Gehirn stammender neurotropher Faktor bedeutet.

2. Verfahren nach Anspruch 1, wobei das differenzierungsinduzierende Kulturmedium Serum enthält.

3. Verfahren nach Anspruch 1, wobei die differenzierten Zellen Neuronen sind und der differenzierungsinduzierende Wachstumsfaktor ciliärer neurotropher Faktor ist.

4. Verfahren nach Anspruch 1, wobei die differenzierten Zellen reife Oligodendrozyten sind und der differenzierungsinduzierende Wachstumsfaktor ciliärer neurotropher Faktor ist.

5. Verfahren nach Anspruch 1, wobei die differenzierten Zellen Astrozyten sind, der differenzierungsinduzierende Wachstumsfaktor ciliärer neurotropher Faktor ist, und das differenzierungsinduzierende Kulturmedium im Wesentlichen frei von Serum ist.

6. Verfahren nach Anspruch 1, wobei die differenzierten Zellen Neuronen sind und der differenzierungsinduzierende Wachstumsfaktor aus dem Gehirn stammender neurotropher Faktor ist.

7. Verfahren nach Anspruch 1, wobei die differenzierten Zellen Neuronen sind und der differenzierungsinduzierende Wachstumsfaktor Retinsäure ist.

8. Verfahren nach Anspruch 1, wobei die differenzierten Zellen Neuronen sind und der differenzierungsinduzierende Wachstumsfaktor bFGF ist.

9. Verfahren nach einem der vorangehenden Ansprüche, wobei das Proliferieren der mindestens einen vorher erhaltenen neuralen Säugetier-Stammzelle in Schritt a) außerdem folgende Schritte aufweist:
i) Bewahren der erhaltenen neuralen Stammzellen in einem ersten proliferationsinduzierenden Kulturmedium, das bFGF enthält; und
ii) Vermehren der bewahrten neuralen Stammzellen in einem zweiten proliferationsinduzierenden Kulturmedium, das EGF und bFGF enthält, um neurale Vorläuferzellen hervorzubringen.

## Revendications

1. Procédé de préparation de cellules différenciées à partir de cellules souches neurales comprenant les étapes consistant à :
a) faire proliférer au moins une cellule souche neurale isolée capable de produire une descendante en mesure de se différencier en neurones, astrocytes et oligodendrocytes en suspension dans un milieu de culture sans sérum comprenant le facteur de croissance épidermique (EGF) comme mitogène, qui permet la prolifération d'au moins une cellule souche pour produire des cellules précurseurs neurales, et
b) différencier les cellules précurseurs neurales en éliminant le mitogène et mettre en culture lesdites cellules précurseurs dans un milieu de culture sans EGF, induisant une différenciation, afin de produire des cellules différenciées, ledit milieu étant préparé avec un facteur de croissance exogène choisi dans le groupe constitué de CNTF, bFGF, BDNF et de l'acide rétinoïque, dans lequel CNTF signifie facteur neurotrophique ciliaire, bFGF signifie facteur de croissance de fibroblastes basiques et BDNF signifie facteur neurotrophique dérivé du cerveau.

2. Procédé selon la revendication 1, dans lequel le milieu de culture induisant une différenciation contient du sérum.

3. Procédé selon la revendication 1, dans lequel les cellules différenciées sont des neurones et le facteur de croissance induisant une différenciation est un facteur neurotrophique ciliaire.

4. Procédé selon la revendication 1, dans lequel les cellules différenciées sont des oligodendrocytes matures et le facteur de croissance induisant une différenciation est un facteur neurotrophique ciliaire.

5. Procédé selon la revendication 1, dans lequel les cellules différenciées sont des astrocytes, le facteur de croissance induisant une différenciation est un facteur neurotrophique ciliaire et le milieu de culture induisant une différenciation est sensiblement dénué de sérum.

6. Procédé selon la revendication 1, dans lequel les cellules différenciées sont des neurones et le facteur de croissance induisant une différenciation est un facteur neurotrophique dérivé du cerveau.

7. Procédé selon la revendication 1, dans lequel les cellules différenciées sont des neurones et le facteur de croissance induisant une différenciation est l'acide rétinoïque.

8. Procédé selon la revendication 1, dans lequel les cellules différenciées sont des neurones et le facteur de croissance induisant une différenciation est le bFGF.

9. Procédé selon l'une quelconque des revendications précédentes, dans lequel la prolifération de l'au moins une cellule souche neurale mammifère obtenue précédemment dans l'étape a) comprend en outre les étapes consistant à :
i) maintenir les cellules souches neurales obtenues dans un premier milieu de culture induisant la prolifération contenant du bFGF ; et
ii) faire proliférer les cellules souches neurales maintenues dans un second milieu de culture induisant la prolifération contenant de l'EGF et du bFGF pour produire des cellules précurseurs neurales.
